(19) Europäisches Patentamt
European Patent Office
Office européen des brevets

(11) **EP 2 383 570 A2**

(12) **EUROPEAN PATENT APPLICATION**

(43) Date of publication:
**02.11.2011 Bulletin 2011/44**

(51) Int Cl.:
**G01N 33/487** (2006.01)

(21) Application number: **11164207.0**

(22) Date of filing: **28.04.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **30.04.2010 JP 2010105683**

(71) Applicant: **Arkray, Inc.**
**Minami-ku**
**Kyoto-shi**
**Kyoto 601-8045 (JP)**

(72) Inventors:
• **Araki, Toshihiko**
**Kyoto-shi, Kyoto 602-0008 (JP)**
• **Nishino, Susumu**
**Kyoto-shi, Kyoto 602-0008 (JP)**

(74) Representative: **MacDougall, Alan John Shaw**
**Mathys & Squire LLP**
**120 Holborn**
**London**
**EC1N 2SQ (GB)**

(54) **Measuring device, measuring method, and program**

(57) A measuring device (1) is provided, comprising a measurement section (5) for measuring a specific constituent in a biological sample, an input accepting section (2) for accepting an input of an estimated value of subject's measurement result, a coincidence degree determining section (61) for referencing data of an estimated value whose input is accepted at the input accepting section (2), and determining a degree of coincidence of a measured value obtained at the measurement section and the estimated value, and output sections (3, 4) for outputting a determined result obtained at the coincidence degree determining section (61).

Fig.1

## Description

[Technical Field]

This invention relates to a measuring device, such as blood glucose meter for self-examination, for measuring a concentration of a specific constituent in subject's own biological sample.

[Background Art]

A patient who has a lifestyle disease or the like routinely needs to understand biological constituent states (such as a concentration) related to such his own disease for understanding his disease state or managing his medication. For understanding biological constituent states, a mobile measuring device is commonly used.
For example, diabetic patients need to check their blood glucose levels for dietary and medication managements on a daily basis. Therefore, the diabetic patients are required to carry blood glucose meters with them to measure their blood glucose levels.
Recently, it has been thought to accustom the patients to measurement of biological constituents or provide an incentive to manage the biological constituent states, by the addition of various new functions on these measuring devices.
For example, the system has been developed, where a doctor sets a target value of patient's blood glucose level, and points are given through a network if a blood glucose level measured with a blood glucose meter is close to the preset target value. In such a system, it has also been proposed to introduce the function that a patient can preliminary send an estimated value of his blood glucose level and points are given through a network if a measured blood glucose level is close to this estimated value (Japanese Unexamined Patent Application Publication No. 2003-6334).
There are also some cases where people other than patients need to understand their biological constituent states. It has been told that, for example, it is effective for athletes to understand their lactate levels after exercises for their training management. Thus, it is recommended that athletes should carry lactate meters with them to measure their lactate levels after exercises.

[Summary of the Invention]

High-frequency measurements for long periods place no small burden on subjects because measurements of biological constituents involve obtaining biological samples, typically such as blood.
It will be useful for subjects as well as managers, such as doctors and trainers who manage the measured value if the number of these measurements can be reduced.
For this reason, it is desirable that subjects develop abilities to understand their biological constituent states from their physical shapes, lifestyles, contents or amounts of exercises, etc., without measuring their biological constituent states.
If subjects can develop these abilities, it will be expected not only to decrease the number of measurements as described above but also to properly manage diet, medication, or training by themselves.
In fact, some patients with long-term diabetic treatments have naturally developed these abilities, and tended to manage diet and medication properly. According to these experiences, in medical practice, there have been growing needs to train the patients who started using blood glucose meters to develop such abilities early.
An issue of the present invention is to provide a measuring device for having functions for helping a subject to develop an ability early to understand a concentration of a specific constituent for the purpose of measurement in his biological sample as described above, and measuring a specific constituent in a biological sample.
This invention for addressing the above issue is a measuring device (hereinafter referred to as "the measuring device of this invention") for measuring a concentration of a specific constituent in subject's own biological sample, comprising a measurement section for measuring a concentration of the specific constituent in a biological sample, an input accepting section for accepting an input of an estimated value of subject's measurement result, a coincidence degree determining section for referencing data of an estimated value whose input is accepted at the input accepting section, and determining a degree of coincidence of a measured value obtained at the measurement section and the estimated value, and an output section for outputting a determined result obtained at the coincidence degree determining section.
The above biological sample includes, but not particularly limited to, blood, urine, sweat, or the like, which is easy to obtain by himself, and the aforementioned specific constituent is the one whose state, such as a concentration in the biological sample, can be associated with a physical condition. The physical condition includes a disease, fatigue caused by training, etc. Specifically, it includes glucose, cholesterol, and triglyceride related to lifestyle diseases including diabetes, obesity, hypercholesterolemia, etc., and lactic acid related to fatigue caused by training. A subject is usually a diabetic patient if the above specific constituent is glucose, a subject is usually an athlete if the above specific constituent is lactic acid, a subject is usually a hypercholesterolemic patient if the above specific constituent is cholesterol, and a subject is usually an obese patient if the above specific constituent is triglyceride.

These measuring devices allow subjects to estimate a measurement result when the subjects routinely perform the measurements of specific constituents, and help the subjects to develop abilities early to understand a concentration of such specific constituents by training.

In a preferred form of the measuring device of this invention, the device further comprises an output data storage section for storing a plurality of output data whose output form is changed depending on a degree of coincidence of the measured and estimated values, and an output data processing section for reading out output data depending on the determined degree of coincidence of the measured and estimated values from the output data storage section, and making the output sections output it.

This form of the measuring device can notify a subject of the degree of coincidence easily. Therefore, it is particularly useful if a subject is an elderly person or a child.

The output data is selected from, for example, image data, character data, and audio data.

In a preferred form of the measuring device of this invention, the device further comprises an instruction data processing section for making the output section output instruction data of instructing to input an estimated value in case of performing a measurement with the measurement section without accepting an input of the estimated value.

This form of the measuring device provides its chance to urge a subject to input an estimated value if he performs a measurement without inputting an estimated value beforehand, and allows a subject to perform an estimation training more certainly.

The present invention for addressing the above issue is a measuring method (hereinafter referred to as "the measuring method of this invention") for measuring a concentration of a specific constituent in a biological sample supplied by a subject, including a measuring step of measuring a concentration of the specific constituent in a biological sample, an input accepting step of accepting an input of an estimated value of subject's measurement result, a coincidence degree determining step of referencing data of an estimated value whose input is accepted in the input accepting step, and determining a degree of coincidence of a measured value obtained in the measuring step and the estimated value, and an output step of outputting a determined result obtained in the coincidence degree determining step.

In a preferred form of the measuring method of this invention, the method further includes an output data processing step of reading out an output data depending on the determined degree of coincidence of the measured and estimated values from output data storage section for storing a plurality of output data whose output form is changed depending on the degree of coincidence, and outputting it, after finishing the coincidence degree determining step.

In a preferred form of the measuring method of this invention, the method further includes an instruction data processing step of outputting instruction data of instructing to input an estimated value in case of performing the measuring step without performing the input accepting step.

In a preferred form of this invention, one device performs a series of steps from input acceptation to output as described above, not through a network.

The present invention also provides a program being read into a measuring device, comprising a computer, for measuring a concentration of a specific constituent in subject's own biological sample, the program (hereinafter referred to as "the program of this invention") for making the computer execute an input accepting step of accepting an input of an estimated value of subject's measurement result, and a coincidence degree determining step of referencing data of an estimated value whose input is accepted in the input accepting step, and determining a degree of coincidence of a measured value of concentration of the specific constituent in a biological sample and the estimated value.

In a preferred form of the program of this invention, the program further makes the computer execute an output data processing step of reading out output data depending on the determined degree of coincidence of the measured and estimated values from an output data storage section for storing a plurality of output data whose output form is changed depending on the degree of coincidence, and outputting it, after finishing the coincidence degree determining step.

In a preferred form of the program of this invention, the program further makes the computer execute an instruction data processing step of outputting instruction data of instructing to input an estimated value in case of performing the measurement of concentration of a specific constituent without performing the input accepting step.

The present invention also provides a computer-readable storage media of storing the program of this invention.

A computer-readable storage media herein refers to the storage media which can accumulate information, such as data and program, with electrical, magnetic, optical, mechanical, or chemical effects, and read it out from a computer.

The measuring device and method of this invention helps a subject to develop an ability early to understand a concentration of a specific constituent on a daily basis without measuring a concentration of a specific constituent for the purpose of measurement in his biological sample. The measuring device and method of this invention is useful for dietary and medication managements for patients with lifestyle diseases, particularly diabetic patients, and training managements for athletes.

[Brief Description of the Drawings]

FIG. 1 is a schematic diagram showing an appearance of one form of the measuring device of this invention.
FIG. 2 is a schematic diagram showing functions of the measuring device in FIG. 1.
FIG. 3 is a flowchart showing a low of a measurement with the measuring device in FIG. 1.

[Mode for Carrying out the Invention]

Hereinafter, the embodiments of this invention are described with reference to the drawings.

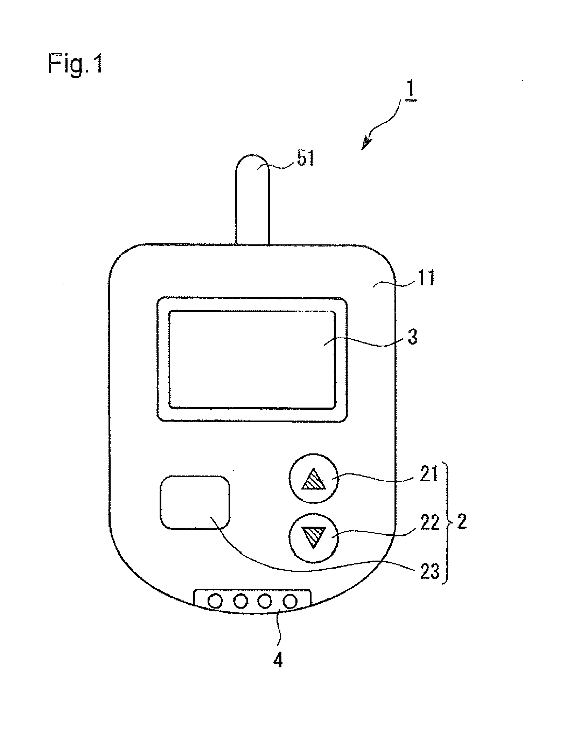

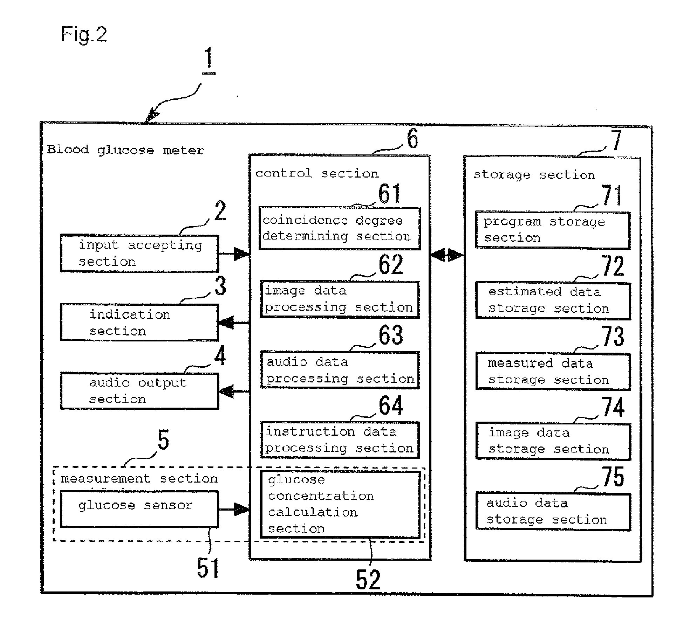

FIG. 1 is a schematic diagram showing an appearance of a blood glucose meter 1 of the measuring device of this invention, and FIG. 2 is a block diagram showing functions of the blood glucose meter 1.
The blood glucose meter 1 is made of a measuring equipment body 11 1 and a glucose sensor 51 removable from the measuring equipment body 11. The glucose sensor 51 is inserted into a slit (not shown) of the measuring equipment body 11, and is removed from the measuring equipment body 11 after being used for a measurement. In this embodiment, the glucose sensor 51 is also configured to be turned on by an insertion of the glucose sensor 51 into the slit.
The measuring equipment body 11 has an input accepting section 2, an indication section 3 and an audio output section 4 as output sections, a control section 6, and a storage section 7. The glucose sensor 51 has a publicly known configuration, and contains a blood spot application section for dispensing a drop of blood, an enzyme membrane for protecting enzymes of reacting with glucose, an electrode for detecting oxidation-reduction potentials, a micro flow path (not shown) for supplying blood dispensed at the blood spot application section to a position of contacting with the enzyme membrane, and the like.

The input accepting section 2 is for accepting an input of an estimated value of a measurement result by a subject, and includes a button, a touch panel, a microphone, and the like. In this embodiment, an increase button (upper button) 21 for increasing a numeric value, a decrease button (lower button) 22 for decreasing a numeric value, and a decision button 23 for fixing an input are used on the basis of predetermined numeric values, but their configurations such as the number, are not particularly limited as long as they can accept an input of an estimated value. If the upper button 21, the lower button 22, and the decision button 23 are used, the upper button 21 and the lower button 22 can be configured so that, for example, the numeric value (mg/dL) displayed on the indication section 3 may be incremented by 10 every time the upper button is pushed, and may be decremented by 10 every time the lower button is pushed, and the decision button 23 can be configured to enable fixing an input. It is noted that it is desirable the configuration of the input accepting section 2 should be as simple as possible because many of the diabetic patients are elderly people. Seen from this standpoint, it is also desirable that, for example, the input accepting section 2 should be configured without the decision button 23 to fix an input of an estimated value when a measurement is performed at a measurement section 5 as described below.

The indication section 3 is one form of the output sections in this invention, and an indication or a display which indicates a measured value obtained at the measurement section 5 as described below, a determined result obtained at a coincidence degree determining section 61, and information of supporting operation of the blood glucose meter 1 by a subject. In this embodiment, an LED-baclclit liquid crystal display is used, but not particularly limited, as long as it can display characters and images. Indication with the indication section 3 is in the forms of characters, such as numbers and messages, and images, such as icons, background colors, and background patterns.

The audio output section 4 is one form of the output sections in this invention, and a thing or a speaker which outputs a measured value obtained at the measurement section 5 as described below, a determined result obtained at the coincidence degree determining section 61, and further a variety of sound for supporting operation of the blood glucose meter 1 by a subject. The audio output section 4 outputs in the forms of languages, such as reading out numbers showing a degree of coincidence, and messages, and music.
The configuration of the audio output section 4 is not necessary in this invention, but it is preferred that this should be included in terms of providing the device which can be utilized by a subject with impaired vision.
In this embodiment, the indication section 3 and the audio output section 4 are employed as the output sections, while the output sections may be merely a thing to output what a subject can recognize with his five senses.

The measurement section 5 includes the above glucose sensor 51 and a glucose concentration calculation section 52.
The glucose sensor 51 includes an enzyme membrane holding glucoseoxidase (GOD) or glucose dehydrogenase (GDH) and an electrode (not shown), and detects an oxidation-reduction current from an enzyme reaction product in glucose when subject's blood touches it.
The glucose concentration calculation section 52 is included in the control section 6 as described below, and calculates a glucose concentration in blood based on calibration curve data (not shown) showing the correlation with the glucose concentration stored in the storage section 7 as described below and a current value.

In this embodiment, it is noted that the glucose measurement section 5 may be a measurement mechanism using a colorimetric method for measuring an amount of dye caused by a reaction of glucose's enzyme reaction product and a specific chromogen, although the case was described where the measurement section 5 is a measurement mechanism using a electrode method. In this case, the measurement section 5 includes a chromogen, an optical measuring equipment, etc.

The control section 6 comprises CPU and RAM, and functions as an output data processing section including the coincidence degree determining section 61, an image data processing section 62, and the audio data processing section 63, an instruction data processing section 64, and the glucose concentration calculation section 52 as described above so that CPU interprets and executes a program developed in RAM. The above program is retained in a program storage section 71 of the storage section 7 as described below. The control section 6 has a clock function and manages input time and date of an estimated value as described below and measurement time and date of the glucose concentration.

The coincidence degree determining section 61 determines a degree of coincidence of an estimated value accepted by the input accepting section 2 and an measured value obtained at the measurement section 5. The degree of coincidence can be determined by obtaining an absolute value calculated by dividing a value subtracting an estimated value from a measured value by a measured value, and further classifying the calculated value into arbitrary ranks as necessary. By way of example only, in this embodiment, it is classified into 3 ranks, equal to or smaller than 0.2 (the coincidence degree is equal to or greater than 80%), greater than 0.2 and equal to or smaller than 0.5 (the coincidence degree is smaller than 80% and equal to or greater than 50%), and greater than 0.5 (the coincidence degree is smaller than 50%).
In addition, for the value used for determining the degree of coincidence, values obtained by subtracting an estimated value from a measured value, and dividing a value subtracting an estimated value from a measured value by a measured value, etc. can be used.
The number of ranks is not also particularly limited, and arbitrary rankings can be applied.

The image data processing section 62, which is one form of the output data processing sections in this invention, reads out image data depending on a determined result obtained at the coincidence degree determining section 61 from an image data storage section 74 as described below, and makes the indication section 3 output it with the measured value data obtained at the measurement section 5.
The audio data processing section 63, which is one form of the output data processing sections in this invention, reads out audio data depending on a determined result obtained at the coincidence degree determining section 61 from an audio data storage section 75 as described below, and makes the audio output section 4 output it. Audio includes a combination of read-out of a measured value data obtained at the measurement section 5 and music.

An instruction data processing section 64 makes the indication section 3 output instruction data of instructing to input an estimated value. The output is performed if the measurement section has measured a value without inputting an estimated value by a subject. The deployment of the instruction data processing section 64 described above can urge a subject to input an estimated value to give its opportunity and certainly have a subject experiencing a training on expectation if a subject dispenses a drop of blood at a glucose sensor without inputting an estimated value previously. Particularly, there are many elderly people in diabetic patients, so the above configuration is useful because there are possibilities for these subjects to dispense a drop of blood right after inserting the glucose sensor 51 into a slit of the measuring equipment body 11 according to their traditional habits of use in blood glucose meters when they start using a new type of blood glucose meters.

It is also preferred that the control section 6 further analyzes a certain period of estimated and measured values stored in an estimated data storage section 72 and a measured data storage section 73 of the storage section 7 as described below, and have a function of evaluating training accomplishments. For example, a function of graphing temporal changes in the degree of coincidence as described above is included.

The storage section 7 has the program storage section 71, the estimated data storage section 72, the measured data storage section 73, and an output data storage section including the image data storage section 74 and the audio data storage section 75.
The program storage section 71 is developed in RAM of the control section 6 as described above, and stores a program executed by CPU.
The estimated data storage section 72 stores estimated value data accepted at the input accepting section 2 as described above, associating the estimated value data with its input time and date. The estimated data storage section 72 holds a certain period of estimated value data.
The measured data storage section 73 stores measured value data obtained at the above measurement section 5, associating the measured value data with its measurement time and date. The measured data storage section 73 holds a certain period of measured value data.
The image data storage section 74 is one form of the output data storage sections in this invention, and stores a plurality of image data whose indication form is changed to be associated with degrees of coincidence of the above estimated and measured values. The image data includes background color data. In this embodiment, case, the image data storage section 74 stores a table which associates 3 types of background color data with 3 ranks of the above degree of coincidence.

The audio data storage section 75 is one form of the output data storage sections in this invention, and stores a plurality of audio data which changes sound to be associated with degrees of coincidence of the above estimated and measured values. In this embodiment case, the audio data storage section 75 stores a table which associates 3 types of music data with 3 ranks of the degree of coincidence as described above.

It is also preferred that the storage section 7 further stores a determined result (degree of coincidence) obtained at the aforementioned coincidence degree determining section 61, associating the determined result with the input time and date of the estimated value or the measurement time and date of the glucose concentration. This configuration allows doctors, etc., to confirm patients' training accomplishments.

In addition, the storage section 7 can store basic information, such as subject information including subject's name, and contact, as being stored in a traditional blood glucose meter.

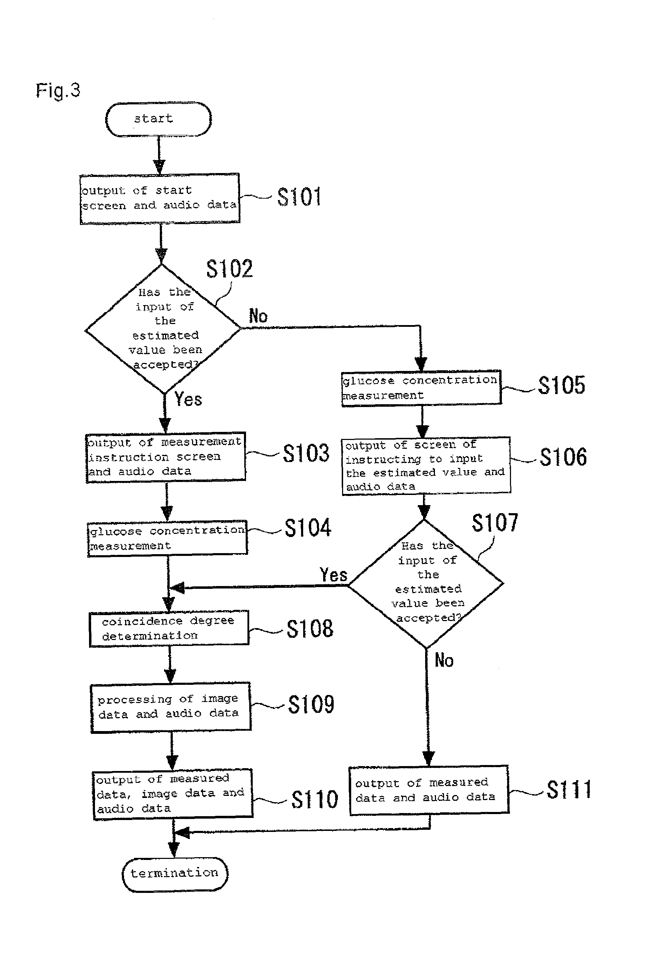

Referring to a flowchart in FIG. 3 below, the behaviors of the blood glucose meter 1 is described.

Firstly, when the glucose sensor 51 is inserted into a slit of the measuring equipment body 11 by a subject, the indication section 3 displays a start screen, and at the same time the audio output section 4 outputs start sound (step S101). The start screen or sound includes, for example, indications of instructing to input an estimated value to the subject.

Next, in the step S102, it is determined whether the input of the estimated value has been accepted at the input accepting section 2 or not. If it is determined that the estimated value has been accepted, the indication section 3 displays a measurement instruction screen of instructing to measure a blood glucose level (dispersing a drop of blood at the glucose sensor 51), and at the same time the audio output section 4 outputs measurement instruction sound of instructing to measure a blood glucose level (step S103). Subsequently, the measurement section 5 waits until the blood contacts the glucose sensor, and then measures a glucose concentration (step S104). After finishing the step S104, the process proceeds to the step S108. The estimated value data whose input was accepted is stored in the estimated data storage section 72 as well as RAM of the control section 6.

On the other hand, in the step S102, if it is determined that the input of the estimated value has not been accepted, the measurement section 5 waits until the blood contacts the glucose sensor, and then measures a glucose concentration (step S105). The measured value data is stored in the measured data storage section 73 as well as RAM of the control section 6. After finishing the step S105, the instruction data processing section 64 makes the indication section 3 output character data and image data of instructing a subject to input an estimated value, and at the same time makes the audio output section 4 output the audio data of instructing to input the estimated value (step S106). After finishing the step S106, the process proceeds to the step S107, and it is determined whether the input of the estimated value has been accepted at the input accepting section 2 or not. If it is judged that the input of the estimated value was accepted in S107, the process proceeds to the step S108.

On the other hand, if it is determined that the input of the estimated value has not been accepted in S107, blank data is stored at the estimated data storage section 72 with being associated with the measurement time and date of the latest glucose concentration, the indication section 3 and the audio output section 4 output the measured value data obtained in the step S105 (step S111), and the process is terminated.

In the step S108, the coincidence degree determining section 61 determines the degree of coincidence of the measured and estimated values. The coincidence degree determining section 61 determines the degree of coincidence of the estimated value data accepted at the input accepting section 2 and the measured value data obtained at the measurement section 5.

The degree of coincidence can be determined by repeating a plurality of judgments depending on the number of ranks and the method of classification. In case of the classification into 3 ranks as described above, for example, the below 2 steps of judgment are included.

It is determined whether the following expression (i) is true or false in the first step.

$$| \, (\text{estimated value - measured value}) \, / \, \text{measured value} \, | \leq x1 \cdots (i)$$

wherein, for example, a numeric value of about 0.2 is set as x1.

If the above expression (i) is true, the image data processing section 62 reads out image data X1 from the image data storage section 74 (step S109), and makes the indication section 3 display it with the measured value data (step S110). In this embodiment, the image data X1 is blue background data, and the indication section 3 displays the measured value on the blue background. The blue background has a high degree of coincidence, which means a good determination result. In this case, the audio data processing section 63 also reads out audio data Y1 from the audio data storage section 75 (step S109), and makes the audio output section 4 output it (step S110). In this embodiment, the audio data Y1 is the one that is a combination of rhythmical music reminiscent of a good result with a phrase of "the measured and estimated values correspond".

If the above expression (i) is false, the process proceeds to the second step to determine whether the following expression (ii) is true or false.

$$\left| (\text{estimated value - measured value}) \,/\, \text{measured value} \right| \leq x2 \cdots \text{(ii)}$$

wherein, for example, a numeric value of about 0.5 is set as x2.

If the above expression (ii) is true, the image data processing section 62 reads out image data X2 from the image data storage section 74 (step S109), and makes the indication section 3 display it with the measured value data (step S110). In this embodiment, the image data X2 is yellow background data, and the indication section 3 displays the measured value on the yellow background. The yellow background does not have high degree of coincidence, which means a normal determination result. In this case, the audio data processing section 63 also reads out audio data Y2 from the audio data storage section 75 (step S109), and makes the audio output section 4 output it (step S110). In this embodiment, the audio data Y2 is the one that is a combination of monotonous music reminiscent of neither good nor bad results with a phrase of "the measured and estimated values are not matched".

If the above expression (ii) is false, the image data processing section 62 reads out image data X3 from the image data storage section 74 (step S109), and makes the indication section 3 display it with the measured value data (step S110). In this embodiment, the image data X3 is red background data, and the indication section 3 displays the measured value on the red background. The red background has a low degree of coincidence, which means a bad determination result. In this case, the audio data processing section 63 also reads out audio data Y3 from the audio data storage section 75 (step S109), and makes the audio output section 4 output it (step S110). In this embodiment, the audio data Y3 is the one that is a combination of sad music reminiscent of bad results with the phrase of "the measured and estimated values are greatly mismatched".

It is noted that, as a variation of the embodiments as described above, the blood glucose meter can be also configured to allow for selecting a function for executing the traditional measurement which measures a blood glucose level without estimating it. In this case, for example, before the step S101, the blood glucose meter may be configured to display, on the indication section 3, a mode selection screen for instructing the selection of an estimating mode to measure a blood glucose level with estimating it and a measuring mode to measure a blood glucose level without estimating it to perform the above processes only if the input of the estimating mode is accepted by the input accepting section 2.

| | |
|---|---|
| 1 | Blood glucose meter |
| 11 | Measuring equipment body |
| 2 | Input accepting section |
| 3 | Indication section |
| 4 | Audio output section |
| 51 | Glucose sensor |

**Claims**

**1.** A measuring device for measuring a concentration of a specific constituent in a biological sample, comprising:

a measurement section for measuring a concentration of the specific constituent in a biological sample,
an input accepting section for accepting an input of an estimated value of subject's measurement result,
a coincidence degree determining section for referencing data of an estimated value whose input is accepted at the input accepting section, and determining a degree of coincidence of a measured value obtained at the measurement section and the estimated value, and
an output section for outputting a determined result obtained at the coincidence degree determining section.

**2.** The measuring device of claim 1, further comprising:

an output data storage section for storing a plurality of output data whose output form is changed depending on a degree of coincidence of the measured and estimated values, and
an output data processing section for reading out output data depending on the determined degree of coincidence of the measured and estimated values from the output data storage section, and making the output section output it.

3. The measuring device of claim 1 or 2, further comprising an instruction data processing section for making the output section output instruction data of instructing to input an estimated value in case of performing a measurement with the measurements section without accepting an input of an estimated value.

4. The measuring device of any one of claim 1 to 3, wherein the output data is selected from image, character, and audio data.

5. The measuring device of any one of claim 1 to 4, wherein the sample to be examined is blood, and the specific constituent is selected from glucose, lactic acid, cholesterol, and triglyceride.

6. A measuring method for measuring a concentration of a specific constituent in a biological sample supplied by a subject, including:

a measuring step of measuring a concentration of the specific constituent in a biological sample,
an input accepting step of accepting an input of an estimated value of subject's measurement result,
a coincidence degree determining step of referencing data of an estimated value whose input is accepted in the input accepting step, and determining a degree of coincidence of a measured value obtained in the measuring step and the estimated value, and
an output step of outputting a determined result obtained in the coincidence degree determining step.

7. The measuring method of claim 6, further including an output data processing step of reading out output data depending on the determined degree of coincidence of the measured and estimated values from an output data storage section for storing a plurality of output data whose output form is changed depending on the degree of coincidence, and outputting it, after finishing the coincidence degree determining step.

8. The measuring method of claim 6 or 7, further including an instruction data processing step of outputting instruction data of instructing to input an estimated value in case of performing the measuring step without performing the input accepting step.

9. A program being read into a measuring device, comprising a computer, for measuring a concentration of a specific constituent in subject's own biological sample,
the program for making the computer execute

an input accepting step of accepting an input of an estimated value of subject's measurement result, and
a coincidence degree determining step of referencing data of an estimated value whose input is accepted in the input accepting step, and determining a degree of coincidence of a measured value of concentration of the specific constituent in a biological sample and the estimated value.

10. The program of claim 9, for further making the computer execute an output data processing step of reading out output data depending on the determined degree of coincidence of the measured and estimated values from an output data storage section for storing a plurality of output data whose output form is changed depending on the degree of coincidence, and outputting it, after finishing the coincidence degree determining step.

11. The program of claim 9 or 10, for further making the computer execute an instruction data processing step of outputting instruction data of instructing to input an estimated value in case of performing the measurement of concentration of a specific constituent without performing the input accepting step.

12. A computer-readable storage media of storing the program of any one of claim 9 to 11.

Fig.1

Fig.2

**Blood glucose meter** 1

**control section** 6

- **coincidence degree determining section** 61
- **image data processing section** 62
- **audio data processing section** 63
- **instruction data processing section** 64

**input accepting section** 2

**indication section** 3

**audio output section** 4

**measurement section** 5

- **glucose sensor** 51
- **glucose concentration calculation section** 52

**storage section** 7

- **program storage section** 71
- **estimated data storage section** 72
- **measured data storage section** 73
- **image data storage section** 74
- **audio data storage section** 75

Fig.3

```
              ┌──────────┐
              │  start   │
              └──────────┘
                   │
                   ▼
         ┌──────────────────┐
         │output of start   │────── S101
         │screen and audio  │
         │data              │
         └──────────────────┘
                   │
                   ▼
              ╱╲ S102
            ╱    ╲
          ╱ Has the╲
        ╱  input of  ╲
       ╱     the       ╲   No
      ╱   estimated      ╲──────────────┐
       ╲  value been    ╱               │
         ╲ accepted?  ╱                 ▼
           ╲       ╱           ┌──────────────────┐
             ╲   ╱             │glucose           │──── S105
              │ Yes            │concentration     │
              ▼                │measurement       │
    ┌──────────────────┐       └──────────────────┘
    │output of         │──S103          │
    │measurement       │                ▼
    │instruction screen│       ┌──────────────────┐
    │and audio data    │       │output of screen  │──── S106
    └──────────────────┘       │of instructing to │
              │                │input the         │
              ▼                │estimated value   │
    ┌──────────────────┐       │and audio data    │
    │glucose           │──S104 └──────────────────┘
    │concentration     │                │
    │measurement       │                ▼
    └──────────────────┘            ╱╲ S107
              │                   ╱    ╲
              │           Yes   ╱ Has the╲
              │◄──────────────╱  input of  ╲
              ▼               ╱    the       ╲
    ┌──────────────────┐       ╲ estimated  ╱
    │coincidence degree│──S108    ╲value been╱
    │determination     │            ╲accepted?╱
    └──────────────────┘              ╲    ╱
              │                        │ No
              ▼                        │
    ┌──────────────────┐               │
    │processing of     │──S109         │
    │image data and    │               │
    │audio data        │               │
    └──────────────────┘               │
              │                        │
              ▼                        ▼
    ┌──────────────────┐       ┌──────────────────┐
    │output of measured│──S110 │output of measured│──── S111
    │data, image data  │       │data and audio    │
    │and audio data    │       │data              │
    └──────────────────┘       └──────────────────┘
              │                        │
              ▼◄───────────────────────┘
       ┌──────────────┐
       │ termination  │
       └──────────────┘
```

**EP 2 383 570 A2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003006334 A